(19) Europäisches Patentamt
European Patent Office
Office européen des brevets

(11) **EP 2 732 800 B1**

(12) **EUROPÄISCHE PATENTSCHRIFT**

(45) Veröffentlichungstag und Bekanntmachung des Hinweises auf die Patenterteilung:
**06.09.2017 Patentblatt 2017/36**

(51) Int Cl.:
***A61F 13/537*** (2006.01)

(21) Anmeldenummer: **13004250.0**

(22) Anmeldetag: **29.08.2013**

(54) **Verteil- und Weiterleitungsvliesstoff**

Distribution and forwarding nonwoven fabric

Non-tissé de distribution et de transmission

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.11.2012 DE 102012022347**

(43) Veröffentlichungstag der Anmeldung:
**21.05.2014 Patentblatt 2014/21**

(73) Patentinhaber: **Sandler AG**
**95126 Schwarzenbach/Saale (DE)**

(72) Erfinder:
• **Obermoser, Alexander**
**95180 Berg (DE)**

• **Bernhuber, Uwe**
**95032 Hof/Saale (DE)**
• **Höflich, Wolfgang**
**95126 Schwarzenbach/Saale (DE)**

(74) Vertreter: **Grünecker Patent- und Rechtsanwälte PartG mbB**
**Leopoldstraße 4**
**80802 München (DE)**

(56) Entgegenhaltungen:
**EP-A1- 1 067 228**

EP 2 732 800 B1

**Beschreibung**

Verteil- und Weiterleitungsvliesstoff

Bei Hygieneartikeln für die Aufnahme und Speicherung von Körperflüssigkeiten ist eine Grundforderung bei möglichst geringer Dicke des Gesamtaufbaus ein möglichst hohes Speichervermögen für aufzunehmende Körperflüssigkeiten zu erzielen.

In der Vergangenheit gab es verschiedene Ansätze, dies zu erreichen.

Prinzipiell haben absorbierende Aufbauten folgende Bestandteile

- Topsheet als zum Körper hingewandte Lage
- Verteil- und Weiterleitungslage zur raschen Verteilung der auftreffenden Flüssigkeit über die gesamte Fläche des Hygieneproduktes und Weiterleitung an die Speicherlage
- Speicherlage zur Immobilisierung der Flüssigkeitsmenge
- Backsheet als flüssigkeitsdichte Außenlage

In den letzten Jahren ging der Trend immer mehr zu einer Speicherlage, welche aus bis zu 100% superabsorbierenden Polymeren besteht. Die vorgeschaltete Verteil- und Weiterleitungslage hat daher die Aufgabe zum Einen, die Flüssigkeit kontrolliert und dosiert weiter zu leiten, um einem Gelblocking vorzubeugen, zum Anderen muss die Flüssigkeit aber auch über möglichst die ganze Fläche des Hygieneartikels verteilt werden, sodass die Speicherschicht vollständig genutzt wird. Dies auch unter Druckeinwirkung.
Auch aufgrund der neuen Coresysteme (höherer Superabsorberanteil, weniger Zellstoff) ist diese Druckstabilität zur Gewährleistung der Verteilung und Weiterleitung von großer Bedeutung.

Für die Aufnahme und Verteilung von Flüssigkeiten in Hygieneartikeln werden üblicherweise sogenannte "Highloftvliese", kardierte Vliesstoffe, die hauptsächlich aus thermoplastischen Fasern bestehen und mittels Heißluft, aber auch mittels Kalandern thermisch verfestigt sind. Beispiele dafür können der der EP 539703 oder auch der DE 19732039 entnommen werden. Auch chemisch verfestigte, d.h. mittels Bindemitteln verfestigte Vliesstoffe werden dafür eingesetzt. Die US 4935022 beschreibt derartige Vliesstoffe.

Diese Vliesstoffe enthalten dabei Fasern wie beispielsweise Spiralfasern oder Hohlfasern zur Erhöhung der Materialdicke.

Jedoch ist bei hoher statischer und dynamischer Belastung, d.h. hoher Beladung der Windel mit Körperflüssigkeiten und dadurch stark gequollenem Superabsorber, insbesondere auch im Gebrauch einer solchen über Nacht, diese Flüssigkeitsverteilwirkung nicht mehr ausreichend gegeben. Verursacht wird dies durch eine Dickenverminderung des Verteil- und Weiterleitungsvliesstofifes, kurz VUWV.

Aufgabe der vorliegenden Erfindung ist es daher, einen Vliesstoff bereitzustellen, welcher die vorgenannten Nachteile des Standes der Technik vermeidet.

Gelöst wird dies anhand der Merkmale des Anspruches 1, vorteilhafte Ausgestaltungen sind in den Unteransprüchen 2 - 18 genannt.

Die Erfindung betrifft VUWV, wie sie insbesondere als Zwischenschicht zwischen Deckschicht und Flüssigketts-Absorptionsschicht von Hygieneprodukten verwendet werden.

Um für diese Zwecke eingesetzt werden zu können, muss ein VUWV eine gute Flüssigkeitsverteilung in Verbindung mit hoher Aufnahmegeschwindigkeit für die Flüssigkeiten haben. Dies ist notwendig, da sonst große anfallende Flüssigkeitsmengen von der Absorptionsschicht nicht schnell genug aufgenommen werden könnten, was zu einer Leckage des Hygieneartikels führen kann. Weiterhin ist ein gutes Rücknässungsverhalten erforderlich, damit einmal von der Absorptionsschicht aufgenommene Flüssigkeit von dieser nicht wieder abgegeben wird und in Richtung Körper wandert. Das Rücknässungsverhalten wird unter anderem maßgeblich durch die Dicke und Druckstabilität des VUWV beeinflusst.

Die Absorptionsschichten bzw. Absorptionskörper moderner Hygieneprodukte enthalten neben einer Matrix aus hauptsächlich hydrophilen Zellulose-Pulpfasern einen grossen Anteil superabsorbierender, hydrogelbildender Partikel, so dass in den Saugkern eines Hygieneproduktes eintretende Flüssigkeit sehr intensiv gebunden werden kann. Ist bei einem Hygieneprodukt kein Flüssigkeitsverteilvlies vorhanden, so durchdringt die eintretende Flüssigkeit die Deckschicht überwiegend senkrecht, wodurch eine große Flüssigkeitsmenge auf eine relativ kleine Fläche verteilt wird und infolgedessen nur die genau unter dieser Fläche befindlichen superabsorbierenden Partikel zur Flüssigkeitsbindung in Aktion treten können. Die außerhalb dieser Fläche befindlichen superabsorbierenden Partikel bleiben dagegen ungenutzt. Ein großer Flüssigkeitsschwall auf einer kleinen Fläche bringt die von der Flüssigkeit benetzten superabsorbierenden Partikel sehr rasch zum Quellen, wodurch sich in der Außenzone des Saugkerns des Hygieneproduktes eine Gelschicht bildet, welche das weitere Eindringen der Flüssigkeit in das Innere des Saugkerns verhindert.

Diesen Zustand bezeichnet man als "Gel-Blocking". Er verhindert eine entsprechend gute Aufnahme spontan auftretender großer Flüssigkeitsmengen, weshalb man bestrebt ist, eine spontan auftretende große Flüssigkeitsmenge vor dem Eindringen der Flüssigkeit in den Saugkern auf eine möglichst große Fläche zu verteilen, so dass die im Saugkern befindlichen, superabsorbierenden Partikel möglichst komplett ausgenutzt werden und die Flüssigkeitsmenge je Flächeneinheit möglichst gering wird, wodurch erreicht werden soll, dass sich das sogenannte "Gel-Blocking" nicht oder kaum negativ auswirkt. Je höher Anteil an Superabsorbern in der Speicherlage (maximal 100%), desto größer die Gefahr eines Gelblockings.

Es ist weiter bekannt, dass im allgemeinen das Einbringen der Flüssigkeit in Hygieneprodukte in sehr kurzer Zeit und in großer Menge erfolgt, weshalb es erforderlich ist, dass die relativ große Flüssigkeitsmenge nahezu augenblicklich vom Saugkörper aufgenommen und festgehalten wird.

Es muss daher verhindert werden, dass sich unter der Deckschicht ein Flüssigkeitsstau bildet, da sich stauende Flüssigkeit nicht vom Saugkörper aufgenommen werden und aus dem Hygieneprodukt an unerwünschten Stellen herauslaufen würde. In diesem Zusammenhang ist es aus der vorerwähnten EP 0 539 703 A1 bekannt, die Fasern einer Flüssigkeitsverteilschicht mit einer hydrophilen Avivage zu versehen. Diese Avivage kann jedoch ihre Aufgabe des Flüssigkeitstransportes nur so lange erfüllen, wie sie sich tatsächlich auf den Fasern befindet. Allerdings weisen nach der EP 0539703 hergestellte Vliese nur eine geringe Stabilität gegen mechanische Druckbelastungen auf. Dies ist bedingt durch die Verwendung von chemisch inkompatiblen Fasern. Gemäß der EP0539703 wird eine bikomponente Schmelzfaser mit einem Polyethylen-Mantel und einem Polyester-Kern mit einer Polyester-Faser verbunden. Bekanntermaßen baut sich dabei keinerlei Haftung zwischen dem Schmelzmantel der Bikomponentenfaser und der Polyesterfaser auf, es ist lediglich ein mechanisches Anhaften. Bereits bei geringer Belastung reißen die Bindepunkte und das so zusammengesetzte Vlies verliert die Stabilität. In der Folge kann das Vlies keine Flüssigkeitsverteilung- und Weiterleitung mehr gewährleisten und es kommt zur vermehrten Rücknässung. Der Ansatz der DE19732039, hier gleiche Polymere und Kalanderverfestigung zu verwenden, löst das Problem nicht, da ein so hergestelltes Material eine zu geringe Eigendicke aufweist und daher wiederum bei Druckbelastung keine Flüssigkeitsverteilung mehr bewirkt.

Hier setzt die Erfindung an.

Ein erfindungsgemäßes Vlies weist eine im Vergleich zum Stand verbesserte Beständigkeit gegen Druckbelastungen auf. Dabei besteht ein erfindungsgemäßes Vlies, welches als VUWV Verwendung findet, prinzipiell aus zwei Komponenten:

- Matrixkomponente: die Matrix wird Fasern und/oder Filamente mit einem Titer von größer 1,7dtex gebildet. Hauptaufgaben der Matrixkomponente sind ist die Flüssigkeitsverteilung.

- Bindekomponente: die Bindekomponente kann erfindungsgemäß in fasriger Form als Filament, als homopolymere oder bikomponente Stapelfaser vorliegen, einsetzbar sind aber auch pulverförmige Systeme, welche an den Kreuzungspunkten der Matrixkomponenten untereinander die erfindungsgemäßen elastischen Verbindungen ausbilden. Die Hauptaufgabe der Bindekomponente ist die Gewährleistung einer elastischen Verbindungsstelle der Matrixkomponenten, deren mechanische Stabilität bei dauerhafter und mehrfacher Druckbelastung und dadurch die Aufrechterhaltung der Flüssigkeitsverteil- und Weiterleitungswirkung, auch unter Belastung.

Für die Matrixkomponente kommen thermoplastische Polymere aus beispielsweise Polyestern oder Polypropylenen aber auch nicht thermoplastische mit bspw. Polyacryl zum Einsatz, für die Bindekomponente kommen thermoplastische und elastische Eigenschaften aufweisende Polymere, kurz TPE.

Unter der Bezeichnung TPE sind verschiedenste Polymere subsummiert. Verfügbar sind folgende Polymere:

- TPO = Thermoplastische Elastomere auf Olefinbasis, vorwiegend PP/EPDM,
- TPV = Vernetzte thermoplastische Elastomere auf Olefinbasis, vorwiegend PP/EPDM
- TPU = Thermoplastische Elastomere auf Urethanbasis
- TPC = Thermoplastische Polyesterelastomere
- TPS = Styrol-Blockcopolymere
- TPA = Thermoplastische Copolyamide,

(Quelle: http://de.wikipedia.org/wiki/Thermoptastische_Elastomere)

Zur Herstellung derartiger Vliese kommen entweder Extrusionsverfahren oder mechanische Vliesbildungsverfahren zum Einsatz.

Bei der Herstellung mittels Extrusionsverfahren wird zunächst ein herkömmliches Spinnvlies in einem Flächengewichtsbereich von 20 bis 120g/m$^2$, bevorzugt von 40 bis 80g/m$^2$, bei Filamenttitern zwischen 1,7 und 13 dtex, bevorzugt im Bereich von 5 bis 10 dtex, erzeugt. Auf die noch spinnwarmen, die Matrixkomponente bildenden Filamente wird anschließend ein, die Bindekomponente bildender Meltblown-Vliesstoff geblasen, welcher aus einem elastischen, thermoplastischen Polymer gebildet wird. Wichtig ist dabei, dass die im MeltbJown-Verfahren gebildeten Fasern einen Durchmesser haben, der unterhalb derer der Fasern der Matrixkomponente liegt. Nur so ist gewährleistet, dass die Meltblown-Fasern in die durch die Matrixkomponente gebildete Struktur eindringen, sich an den Kreuzungspunkten ablagern und dort erkalten.

Anwendbar ist für die Verfestigung des losen Filamentverbundes auch die Applikation eines pulverförmigen Bindemittels, welches nach dem Schmelzen und Erkalten im Bereich der Filamentkreuzungspunkte erfindungsgemäß elastische Bindepunkte ausbildet.

Des Weiteren und erfindungsgemäß bevorzugt kommen Vliesstoffe aus Stapelfasern zum Einsatz.

Die Matrixkomponente wird dabei von Stapelfasern aus thermoplastischen Polymeren wie Polypropylen, Polyester oder Polyamid aber auch aus nicht thermoplastischen Polymeren wie beispielsweise Polyacryl gebildet. Der zum Einsatz kommende Titerbereich liegt zwischen 1,7 bis 17 dtex, wobei erfindungsgemäß bevorzugt Fasern mit einem Titer von 5 dtex bis 10 dtex verwendet werden. Die die Matrixkomponente bildenden Fasern können auch die elastischen Eigenschaften begünstigende Formen wie beispielsweise Faserquerschnitte mit Hohlräumen in der Mitte oder Spiralkräuselung besitzen. Handelsübliche Viskosefasern können nicht verwendet werden, da diese bei Kontakt mit Flüssigkeit kollabieren, d.h. die Druckbeständigkeit des Vliesstoffes geht verloren.

Die Bindekomponente wird dabei aus Mono- oder Mehrkomponenten-Fasern gebildet, welche einerseits thermoplastisch sind und andererseits ganz oder zumindest in Teilbereichen, z.B. der Mantelbereich bei Bikomponenten-Fasern, thermoplastische und elastische Eigenschaften aufweisen.

Typische Vertreter sind hier beispielsweise bikomponente Kern/Mantel-Stapelfasern, bei welchen der Kern aus einem normalen Polyester gebildet wird.

Der Mantel besteht aus einem thermoplastischen Elastomer, beispielsweise einem Polyurethanelastomer oder Polyesterelastomer. Derartig aufgebaute Fasern sind beispielsweise in der WO9119032 genannt. Für die vorliegende Erfindung wurde eine Faser mit einem Titer von 6.7dtex und einer Stapellänge von 64mm verwendet.

Geeignete Polyurethanelastomere (TPU) sind dabei elastische Thermoplaste, die durch Reaktion eines niedrig schmelzenden Polyols, welches ein Molekulargewicht in der Größenordnung von 500 bis 6000 aufweist, mit einem organischen Diisocyanat, welches ein Molekulargewicht von nicht mehr als 500 aufweist, erhalten werden.

Verwendbare Polyesterelastomere (TPC) sind dabei Polyether/Ester-Blockcopolymere, welche durch Copolymerisation von thermoplastischen Polyestern als harte Segmente und Poly(alkylenoxid)glycolen als weiche Segmente gebildet werden. Erfindungsgemäß bevorzugt werden Fasern mit TPC als elastische Thermoplast-Komponente, da diese speziell bei der Verwendung mit Polyesterfasern als Matrixkomponente eine intensivere Haftung und mechanische Stabilität der Bindepunkte zur Folge haben.

Durch die Verwendung dieser elastischen Polymere ist die Ausbildung der erfindungsgemäßen elastischen, druckbeständigen Bindepunkte gewährleistet.

Bei der Herstellung eines erfindungsgemäßen Vliesstoffes unter Verwendung von Stapelfasern kommen Trocken- oder Naßverfahren zum Einsatz. Die zugrundeliegenden Verfahren können dem im Jahr 2000 bei Wiley VCH-Verlag, Weinheim erschienenen Buch "Vliesstoffe" entnommen werden.

Die nachfolgenden Beispiele beziehen sich, ohne darauf beschränkt zu sein, auf nach einem Trockenverfahren unter Verwendung des Kardierverfahrens hergestellten Stapelfaservliesstoff. Dieser wurde im Anschluss an das Vliesbildungsverfahren thermisch mittels Heißluft verfestigt. Zur Erhöhung der mechanischen Stabilität kann zwischen der Vliesbildung und der thermischen Verfestigung auch noch eine mechanische Verfestigungsstufe geschaltet werden, sodass beispielsweise mittels des Wasserstrahlverfahrens eine für die nachfolgenden Verfahrensgänge ausreichende.Stabilität erreicht wird.

Durch diese mechanische Verwirbelung wird die Ausbildung von potenziellen Kreuzungspunkten begünstigt, was direkt ptopoertional ist zur thermischen Verfestigung. Dies hat eine höhere Druckbeständigkeit zur Folge.

Erfindungsgemäß sind dabei Anteile von 15 bis 80 Gewichts-% der Matrixkomponente in Form von Polyester-Stapelfasern und komplementär dazu 85 bis 20 Gewichts-% Bindekomponenten in Form von Bikomponentenfasern zum Einsatz. Um eine bessere Haftung zu den Matrixfasern zu gewährleisten, wurden erfindungsgemäß bevorzugt bei den Ausführungsbeispielen Fasern mit einem Polyester Kern und einem TPC Mantel eingesetzt.

Die Mischungsverhältnisse der beiden Komponenten zueinander, d.h. der Anteil an Matrix- und an Bindekomponente, richtet sich dabei nach dem Anforderungsprofil für den gedachten Endeinsatzzweck.

Soll beispielsweise der erfindungsgemäße VUWV im Bereich von Baby-Windeln Anwendung finden, so ist schnelle Aufnahme von Flüssigkeiten und gute Verteilung gefordert. Die Rücknässung unter Belastung ist aufgrund der geringen einwirkenden Druckkräfte durch das Baby weniger kritisch zu sehen. Entsprechend höher ist der Anteil der Matrixkomponente, er liegt erfindungsgemäß zwischen 40 und 85 Gewichts-% bei Flächengewichte liegen zwischen 20 und 130 g/m$^2$, bevorzugt zwischen 50 und 100 g/m$^2$

Werden Erwachseneninkontinenzprodukte mit einem erfindungsgemäßen VUWV ausgestattet, so ist das Hauptaugenmerk mehr auf die Flüssigkeitsweiterleitung und Verteilwirkung, sowie die Rücknässung unter Belastung zu legen. Entsprechend reduziert wird der Anteil der Matrixkomponente. Erfindungsgemäß haben sich Mischungen mit einem Anteil von 15-60% Matrixkomponente bewährt. Die Flächengewichte liegen hier zwischen 40 und 150g/m$^2$, be-

vorzugt zwischen 60 und 130 g/m$^2$

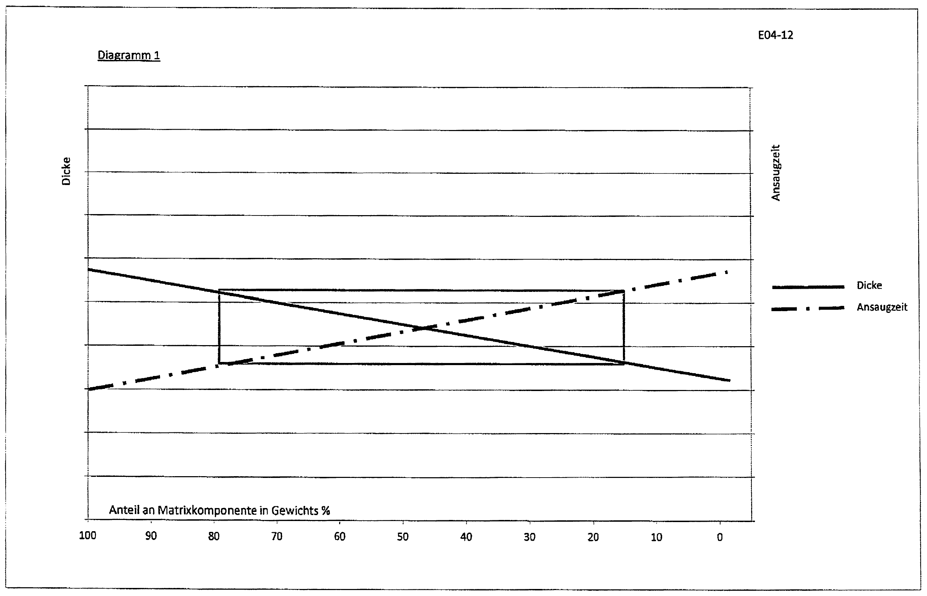

Diesen Zusammenhang soll das Diagramm 1 verdeutlichen. Hier wurde schematisch der Zusammenhang zwischen den Anteilen an Matrixkomponente und der Dicke, sowie der Ansaugzeit des Materials aufgetragen.

Innerhalb des erfindungsgemäß bevorzugten Anteils an Matrixkomponente, d.h. zwischen 15 und 80 Gewichts%, ändern sich die Dicke und die Ansaugzeit nur marginal. Die tatsächlichen Anteile an Matrixkomponente müssen für den jeweiligen Endaufbau optimiert werden, sodass hier nur der ursächliche Zusammenhang aufgezeigt werden.

Anteile von größer 80% Matrixfaser führen zwar zu hoher Dicke, jedoch ist die Ansaugzeit deutlich geringer. Ebenso ist der Höhenverlust stärker ausgeprägt, da entsprechend wenig elastische Verbindungspunkte ausgebildet werden. Bei Anteilen der Matrixkomponente von kleiner 15% ist die Dicke des VUWV, speziell unter Belastung zu gering, sodass die Ansaugzeiten nach oben gehen. Vermehrtes Rücknässen ist die Folge.

In den folgenden Ausführungen wird näher auf erfindungsgemäße Materialien eingegangen, die als VUWV für den Einsatzbereich Babywindeln geeignet sind. Verglichen werden diese mit den Vergleichsbeispielen 1 und 2, dem Stand der Technik entsprechenden VUWV's.

Ergänzend dazu sind in Tabelle 1 die einzelnen erzielten Prüfergebnisse aufgeführt. Die dabei ermittelten Parameter wurden gemäß den nachfolgend genannten Prüfmethoden ermittelt.

Flächengewicht: gemäß WSP 130.1

Dicke: in Anlehnung an WSP 120.6 mit einem Messdruck von 0,5kPa

Ansaugzeit: Eine kommerziell erhältliche Windel des Typs Pampers Maxi Dry, Größe 4+ wird flach auf ein Brett aufgespannt und an den Kanten fixiert, sodass die Windel plan liegt. Anschließend wird das Topsheet und der VUWV vorsichtig entfernt und der Saugkörper freigelegt. Der Saugkörper wird dann mit dem erfindungsgemäß ausgeführten oder dem Stand der Technik entsprechendem VUWV abgedeckt und das originale Topsheet wiederum zur Abdeckung aufgelegt. Abschließend wird auf das Topsheet eine Prüfplatte aufgelegt, welche eine zentrale Öffnung mit darüber liegenden Fülltrichter aufweist. Der Durchmesser der Öffnung und des Trichters beträgt 38mm. Die Platte wird so auf der Windel platziert, dass die Öffnung mittig auf der Windel zu liegen kommt. Anschließend wird die Prüfplatte mit Gewichten beschwert, bis ein Druck von 5100 Pa (+/- 50Pa) auf den Testaufbau wirkt. Zum eigentlichen Test werden dann im Abstand von 300 Sekunden jeweils 70ml einer 0.9% Kochsalzlösung mit einer Fließgeschwindigkeit von von 15ml/s über den Fülltrichter auf den Testaufbau gegeben. Zur Ermittlung der Ansaugzeit wird die Zeit in Sekunden ermittelt, welche zwischen dem Start der Flüssigkeitsaufgabe und dem Versickern des letzten Tropfens einer Flüssigkeitsaufgabe vergeht. Die Flüssigkeitsaufgabe wird dreimal an gleicher Stelle wiederholt, sodass insgesamt 280ml Flüssigkeit in den Testaufbau gegeben werden.

Höhenverlust: Vom zu untersuchenden Material werden quadratische Muster einer Fläche von 100cm$^2$ hergestellt. Anschließend werden so viele Stanzlinge aufeinander gelegt, dass eine Gesamtdicke von ca. 15mm erreicht wird. Ein derartiger Musterstapel wird dann einer Zwick-Prüfmaschine, Typ 1425 zur Ermittlung der Dicke bei verschiedenen Druckbelastungen vorgelegt. Der Musterstapel wird auf eine Grundplatte der Größe 11*11cm gelegt. Anschließend startet eine Druckbelastung mit einem Druckstempel gleicher Größe. Die Verformungs- oder Belastungsgeschwindigkeit beträgt dabei 100mm/min. Bei Erreichen eines Drucks von 0,02kPa, 1,0kPa und 2,0kPa wird jeweils die Höhe des Musterstapels ermittelt. Abschließend erfolgt eine Ermittlung des Höhenverlusts gemäß der Formel:

$$\text{Höhenverlust} = \frac{\text{Stapelhöhe bei 0,02kPa} - \text{Stapelhöhe bei 2kPa}}{\text{Stapelhöhe bei 0,02kPa}} * 100\%$$

Das erfindungsgemäß ausgeführte Vlies 1 stellt ein VUWV dar, welches in einer Babywindel zum Einsatz kommen soll. Das erfindungsgemäß ausgeführte VUWV 1 setzt sich zusammen aus

65 Gewichts-% der Matrixkomponente, hier gebildet aus handelsüblichen Polyethylenterephthalatfasern mit einem Titer von 10 dtex, Stapellänge 64mm, Hohlfaser mit Spiralkräuselung.

35 Gewichts-% der Bindekomponente, hier gebildet aus einer bikomponenten Kern-/ Mantel Stapelfaser, wobei der Kern aus handelsüblichen Polyethylenterephthalat und der Mantel aus einem thermoplastischen Polyesterelastomer-Faser (TPC) besteht. Der Mantel-Anteil beträgt ca. 50% Gewichts% der Gesamtfaser. Der Titer liegt bei 6.0 dtex, die Stapellänge liegt bei 64mm.

Die Vliesbildung geschieht mittels eines üblichen Krempelverfahrens, die anschließende Verfestigung erfolgt mittels Heißluft einer Temperatur von 140°C während ca. 3 Minuten. Das so hergestellte Vlies hat ein Flächengewicht von ungefähr 131 g/m$^2$ bei einer Dicke von 4,18 mm.

Das erfindungsgemäß ausgeführte Vlies 2 stellt ein VUWV dar, welches in einer Erwachsenenwindel zum Einsatz kommen soll. Daher wird mehr Augenmerk auf die mechanische Stabilität gesetzt. Das erfindungsgemäß ausgeführte Vlies 2 setzt sich daher zusammen aus

35 Gewichts-% der Matrixkomponente, hier gebildet aus handelsüblichen Polyethylenterephthalatfasern mit einem Titer von 10 dtex, Stapellänge 64mm, Hohlfaser mit Spiralkräuselung.

65 Gewichts-% der Bindekomponente, hier gebildet aus einer bikomponenten Kern-/ Mantel Stapelfaser, wobei der Kern aus normalem Polyethylenterephthalat und der Mantel aus einem thermoplastischen Polyesterelastomer-Faser (TPC) besteht. Der Mantel-Anteil beträgt ca. 50% Gewichts% der Gesamtfaser. Der Titer liegt bei 6.0 dtex, die Stapellänge liegt bei 64mm.

Die Vliesbildung geschieht mittels eines üblichen Krempelverfahrens, die anschließende Verfestigung erfolgt mittels Heißluft einer Temperatur von 140°C während ca. 3 Minuten. Das so hergestellte Vlies hat ein Flächengewicht von ungefähr 133 g/m$^2$ bei einer Dicke von 3,96mm.

Das Vergleichsbeispiel 1 ist ein Verteilvlies, wie es in Babywindeln des Typs Pampers Maxi Dry, Größe 4+ eingesetzt wird. Es ist ein kardiertes Stapelfaser-Vlies aus 100% Polyesterfasern im Titer von 6,7dtex, welche chemisch, d.h. durch Applikation eines Bindemittels mit einander verbunden wurden. Das so hergestellte Vlies hat ein Flächengewicht von ungefähr 63,7g/m$^2$ bei einer Dicke von 1,43mm

Das Vergleichsbeispiel 2 ist ein Verteilvlies, wie es handelsüblich für Babywindel eingesetzt wird. Bei der Type Slimcore TL1 von Libeltex, Belgien handelt es sich um ein kardiertes Stapelfaser-Vlies aus einem Gemisch von ca. 50Gewichts-% normaler Polyesterfasern im Titer von ca. 4dtex als Matrixkomponente, mit ca. 50% einer bikomponenten Schmelzfaser mit einem Co-Polyester-Mantel und einem Polyester-Kern im Titer von ca. 6dtex. Im Unterschied zur Erfindung bildet das hier verwendete Mantelpolymer keine elastischen Bindestellen aus. Das Vlies des Vergleichsbeispiels 2 hat ein Flächengewicht von ungefähr 59,7 g/m$^2$ bei einer Dicke von 2,39mm.

Betrachtet man nun die Tabelle 1 so kann man folgendes erkennen.

Die beiden erfindungsgemäß ausgeführten VUWV haben bei gleichem Flächengewicht unter definierter Vorlast eine deutlich höhere Dicke. Bei der Ermittlung der Ansaugzeit, wie vorerwähnt findet die Flüssigkeitszugabe unter einer Belastung von 5100Pa +/-50pa statt. Wie der Tabelle 1 zu entnehmen ist, haben beide erfindungsgemäß ausgeführten VUWV eine deutlich geringere Ansaugzeit wie das dem Stand der Technik entsprechende Vergleichsbeispiel 1. Aufgrund des Anteils an Bindekomponente zwischen 35 und 65 Gewichts-% in den erfindungsgemäßen VUWV's haben so ausgeführte Materialien eine geringere Neigung zur Kompression unter Belastung. Man sieht, dass auch nach Aufgabe von insgesamt 280ml Testflüssigkeit noch keine signifikante Zunahme der Ansaugzeit vorliegt, auftreffende Flüssigkeit wird weiterhin angesaugt, in den durch die Vliesstruktur gebildeten Hohlräumen verteilt und zur Immobilisierung an die Speicherschicht weitergeleitet. Die Verteil- und Weiterleitungseigenschaften werden nur wenig beeinträchtigt. Verringert man den Anteil an Bindekomponente auf ca. 35 Gewichts-% wie im erfindungsgemäß ausgeführten VUWV 2, ist die Ausgangsdicke etwas geringer, die Ansaugzeit erhöht sich allerdings nur leicht. Das Vergleichsbeispiel 1 zeigt jedoch in jedem Fall höhere Ansaugzeiten, die sich durch die fehlende Rückstellkraft erklären lassen. Für den Einsatzbereich in Windeln ist eine schnelle Ansaugzeit notwendig, da sich dadurch die Gefahr eines Flüssigkeitsaustritts an den Seiten der Windel verringert. Letzteres ist ein wichtiges Kriterium für Tragesituation, in welchen unkontrollierte Bewegungen bzw. Seitenlage auftreten, wie beispielsweise im Schlaf.

Zur Verdeutlichung dieses Zusammenhangs soll die Tabelle 2 dienen.

Hier wurden die sich bei Druckbelastung eines Musterstapels resultierenden Dicken ermittelt. Wie man deutlich erkennen kann, erfahren Stapel aus erfindungsgemäß ausgeführten VUWV bei Belastung von 2kPa einen im Vergleich zur Ausgangsdicke, welche bei Belastung von 0,02kPa ermittelt wurde, geringeren Höhenverlust. Dieser liegt beim erfindungsgemäß ausgeführten VUWV 1 bei 17,1%, erfindungsgemäß ausgeführten VUWV 2 bei 18,1%. Betrachtet man dagegen die beiden Beispiele aus dem Stand der Technik, so liegt deren Höhenverlust bei 48% für das Vergleichsbeispiel 1 und bei 67% für das Vergleichsbeispiel 2.

Das Vergleichsbeispiel 2 ist hier von besonderem Interesse, da dies mittels ähnlicher Fertigungstechnologie und bis auf die Verwendung von thermoplastischen und elastischen Polymeren in der Bindekomponente der erfindungsgemäß hergestellten VUWV aus vergleichbaren Rohstoffen erzeugt wurde. Das Vergleichsbeispiel 2 zeigt den größten Höhenverlust innerhalb der Messreihe, was bedeutet, dass bei Belastung die Struktur durch aufbrechende Verbindungspunkte kollabiert, eine geregelte Flüssigkeitsaufnahme und Verteilung ist nicht mehr möglich. Dies erkennt man auch bei dem Vergleichsbeispiel 1, wobei hier der Höhenverlust 48%. Betrachtet man dazu die Tabelle 1 erkennt man wiederum die Zunahme der Ansaugzeiten, welche mit bei vierter Flüssigkeitsaufgabe bei 46 Sekunden liegen.

Erfindungsgemäß ausgeführte VUWV haben aufgrund ihrer besseren mechanischen Stabilität, hier ein um den Faktor 2 bis 3 geringerer Höhenverlust im Vergleich zum Stand der Technik, einen deutlichen Vorteil. Sie gewährleisten auch in ungünstigen Anwendungssituationen eine gute Flüssigkeitsverteilung und Weiterleitung dieser an die Sauglage zur Immobilisierung.

Tabelle 1: Vergleich der Ansaugzeiten

| Parameter | Einheit | Erfindungsgemäß ausgeführtes VUWV 1 Bezeichnung sawaflor 99DI050201 | Erfingdungsgemäß ausgeführtes VUWV 2 Bezeichnung sawaflor 99DI050202 | Vergleichsbeispiel 1 nach dem Stand der Technik |
|---|---|---|---|---|
| Aufbau / Zusammensetzung des VUWV im Test | | **Bindekomponente:** 35 % PET/PET-Bikomponentenfaser 6,7dtex,64mm **Matrixkomponente:** 65% PET 10dtex/ 164mm,spiralgekräuselte Hohlfaser, | **Bindekomponente:** 65 % PET/PET-Bikomponentenfaser 6.7dtex,64mm **Matrixicomponente:** 35% PET 10dtex /64mm,spiralgekräuseilte Hohlfaser. | Originaler VUWV der Testwindel, doppelt aufeinander gelegt. Material ist ein bindemittelverfestigtes Vlies aus Polyester-Stapelfasern mit 6,7dtex |
| Flächengewicht | $g/m^2$ | 131,0 | 133,0 | 127,4 (zwei Lagen übereinander gelegt) |
| Dicke | mm | 4,18 | 3,96 | 2,86 |
| Ansaugzeit bei erster Flüssigkeitsaufgabe | s | 13 | 15 | 20 |
| Ansaugzeit bei zweiter Flüssigkeitsaufgabe | s | 11 | 14 | 29 |
| Ansaugzeit bei dritter Flüssigkeitsaufgabe | s | 12 | 16 | 38 |
| Ansaugzeit bei vierter Flüssigkeitsaufgabe | s | 13 | 18 | 46 |

Tabelle 2: Vergleich der Höhenverluste

| Parameter | Einheit | Erfindungsgemäß ausgeführtes VUWV 1 Bezeichnung sawaflor 99DI050201 | Erfindungsgemäß ausgeführtes VUWV 2 Bezeichnung sawaflor 99DI050202 | Vergleichsbeispiel 1 nach dem Stand der Technik Bezeichnung: VUWV der Testwindel | Vergleichsbeispiel 2 nach dem Stand der Technik Bezeichnung: Slimcore TL 1 |
|---|---|---|---|---|---|
| Herstell- / Verfestigungsart | | Stapelfaservlies, thermisch mittels Heißluft verfestigt | Stapelfaservlies, thermisch mittels Heißluft verfestigt | Stapelfaservlies, chemisch mit Bindemittel verfestigt. | Stapelfaservlies, thermisch mittels Heißluft verfestigt |
| Zusammensetzung des VUWV | | **Bindekomponente:** 35 % TPC/PET-Bikomponentenfaser 6,7dtex,64mm **Matrixkomponente:** 65% PET 10dtex /64mm, spiralgekräuselte Hohlfaser, | **Bindekomponente:** 65 % TPC/PET-Bikomponentenfaser 6,7dtex,64mm **Matrixkomponente:** 35% PET 10dtex/ 64mm, spiralgekrauselte Hohlfaser, | **Bindekomponente:** Auskondensiertes Bindemittel **Matrixkomponente:** PET-Fasern 6,7dtex | **Bindekomponente:** 50% Co-PET/PET-Bikomponentenfasern **Matrixkomponente:** 50% PET-Fasern 6,7dtex |
| Stapelgewicht | 9 | 3,92 (drei Lagen mit je ca. 130 g/m$^2$ übereinander gelegt) | 3,56 (drei Lagen mit je ca. 118 g/m$^2$ obereinander gelegt) | 3,90 (sechs Lagen mit je ca. 65 g/m$^2$ übereinander gelegt) | 3,58 (sechs Lagen mit je ca. 60 g/m$^2$ Übereinander gelegt) |
| Stapelhöhe bei 0,02kPa Vorlast | mm | 13,00 | 11,96 | 14,22 | 14,39 |
| Stapelhöhe bei 1,0kPa Vorlast | mm | 11,50 | 10,59 | 8,61 | 5,46 |
| Stapelhöhe bei 2,0kPa Vorlast | mm | 10,78 | 9,80 | 7,27 | 4,65 |
| Höhenverlust (0,02kPa zu 2kPa) | % | 17,1 | 18,1 | 48,9 | 67,7 |

**Patentansprüche**

1. Verteil- und Weiterleitungsvliesstoff für persönliche Hygieneprodukte, umfassend eine Matrixkomponente und eine elastische Verbindungsstellen ausbildende Bindekomponente welche zumindest teilweise aus einem elastischen Thermoplasten besteht
**dadurch gekennzeichnet,**
**dass** der Anteil der Matrixkomponente, die von Fasern und/oder Filamenten mit einem Titer von grösser als 1,7 dtex gebildet wird, 15 bis 80 Gewichts-% beträgt und komplementär dazu der Anteil der Bindekomponente 85 bis 20 Gewichts-% beträgt.

2. Verteil- und Weiterleitungsvliesstoff gemäß Anspruch 1,
**dadurch gekennzeichnet, dass**
die Bindekomponente

   - aus elastischen, thermoplastischen Schmelzklebepulvern und/oder Granulaten oder
   - aus einem elastischen und thermoplastischen Extrusionsvliesstoff oder
   - aus bikomponenten Schmelzfasern gebildet wird, deren schmelzbare Anteile aus dem elastischen Thermoplasten

   gebildet wird.

3. Verteil- und Weiterleitungsvliesstoff gemäß Anspruch 1,
**dadurch gekennzeichnet, dass**

   - der Verteil- und Weiterleitungsvliesstoff mittels eines Trockenverfahrens aus Stapelfasern hergestellt wird,
   - die Matrixkomponente dabei aus homopolymeren Synthese-Stapelfasern gebildet ist, die thermoplastische oder duroplastische Eigenschaften aufweisen,
   - die Bindekomponente dabei aus bikomponenten Schmelzfasern gebildet wird, deren schmelzbare Anteile aus elastischen Thermoplasten bestehen und
   - der Vliesstoff thermisch mittels Heißluftverfestigung und mechanisch mittels Vernadelung oder Wasserstrahlvernadelung gebunden ist.

4. Verteil- und Weiterleitungsvliesstoff gemäß Anspruch 2 oder 3
**dadurch gekennzeichnet, dass**

   - die bikomponenten Schmelzfasern Kern/Mantel Struktur oder Side-by-Side Struktur aufweisen.

5. Verteil- und Weiterleitungsvliesstoff gemäß Anspruch 1,
**dadurch gekennzeichnet, dass**

   - das Flächengewicht zwischen 20 und 150 g/m$^2$ liegt.

**Claims**

1. Nonwoven fabric for distribution and transferring for personal hygiene products, comprising a matrix component and a resilient binding component forming connection points, which binding component is composed of a resilient thermoplastic polymer at least in part,
**characterised in that**
the proportion of matrix component, which is formed of fibres and/or filaments having a titre of more than 1.7 dtex, is 15 to 80 wt.%, and, complementarily thereto, the proportion of binding component is 85 to 20 wt.%.

2. Nonwoven fabric for distribution and transferring according to claim 1,
**characterised in that**
the binding component is formed of

   - resilient, thermoplastic holt-melt adhesive powders and/or granulates, or
   - a resilient and thermoplastic extrusion nonwoven fabric, or

- bicomponent fusible fibres, the fusible constituents of which are made of the resilient thermoplastic polymer.

3. Nonwoven fabric for distribution and transferring according to claim 1,
   **characterised in that**

   - the nonwoven fabric for distribution and transferring is produced from staple fibres by means of a dry process,
   - the matrix component is formed of homopolymer synthetic staple fibres which have thermoplastic or thermosetting properties,
   - the binding component is formed of bicomponent fusible fibres, the fusible constituents of which are composed of resilient thermoplastic polymers, and
   - the nonwoven fabric is thermally bonded by means of hot-air bonding and mechanically bonded by means of needling or water-jet needling.

4. Nonwoven fabric for distribution and transferring according to either claim 2 or claim 3,
   **characterised in that**

   - the bicomponent fusible fibres have a core/sheath structure or a side-by-side structure.

5. Nonwoven fabric for distribution and transferring according to claim 1,
   **characterised in that**

   - the weight per unit area is between 20 and 150 g/m$^2$.

**Revendications**

1. Non-tissé de répartition et de transmission pour des produits d'hygiène personnels, comprenant un composant formant matrice, et un composant formant liant, qui réalise des points de liaison élastiques et est constitué au moins partiellement d'un thermoplastique élastique,
   **caractérisé en ce que**
   la fraction du composant formant matrice, qui est constitué de fibres et/ou de filaments avec un titre plus grand que 1,7 dtex, est d'une valeur de 15 à 80% en poids, et la fraction du composant formant liant est, de manière complémentaire, d'une valeur de 85 à 20% en poids.

2. Non-tissé de répartition et de transmission selon la revendication 1,
   **caractérisé en ce que**
   le composant formant liant est constitué

   - de poudres et/ou de granulés de colle fusible thermoplastique et élastique, ou bien
   - d'un non-tissé d'extrusion élastique et thermoplastique, ou bien
   - de fibres fusibles bi-composant, dont les fractions fusibles sont formées par les thermoplastiques élastiques.

3. Non-tissé de répartition et de transmission selon la revendication 1,
   **caractérisé en ce que**

   - le non-tissé de répartition et de transmission est fabriqué à partir de fibres courtes, moyennant un procédé à sec,
   - le composant formant matrice est ici constitué de fibres courtes de synthèse, homopolymères, qui présentent des propriétés thermoplastiques ou thermodurcissables,
   - le composant formant liant étant ici constitué de fibres fusibles, bi-composant, dont les fractions fusibles sont constituées de thermoplastiques élastiques, et
   - le non-tissé étant lié thermiquement par une solidification par air chaud, et mécaniquement par aiguilletage ou aiguilletage au jet d'eau.

4. Non-tissé de répartition et de transmission selon la revendication 2 ou la revendication 3,
   **caractérisé en ce que**

   - les fibres fusibles bi-composant présentent une structure âme/enveloppe, ou bien une structure dite side-by-side à disposition côte à côte.

**5.** Non-tissé de répartition et de transmission selon la revendication 1,
**caractérisé en ce que**
le poids par unité de surface ou grammage se situe entre 20 et 150 g/m$^2$.

Diagramm 1

E04-12

Ansaugzeit

Dicke

Dicke — Ansaugzeit

Anteil an Matrixkomponente in Gewichts %

0   10   20   30   40   50   60   70   80   90   100

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 539703 A
- DE 19732039
- US 4935022 A
- EP 0539703 A1
- EP 0539703 A
- WO 9119032 A